# EUROPEAN PATENT APPLICATION

(11) **EP 3 466 932 A1**
(43) Date of publication of application: **10.04.2019**
(21) Application number: 17806528.0
(22) Date of filing: 25.05.2017
(51) Int. Cl.: C07D 239/26, C07D 241/12

(54) **PRODUCTION METHOD FOR BICYCLIC NITROGEN-CONTAINING HETEROCYCLIC COMPOUND**

(30) Priority: 31.05.2016 JP 2016109017
(71) Applicant: Kobelco Eco-Solutions Co., Ltd, Kobe-shi, Hyogo 651-0072 (JP)
(72) Inventor: MURAKAMI Yoshiaki, Kobe-shi Hyogo 651-2241 (JP); FUKUSHIMA Miyuki, Kobe-shi Hyogo 651-2241 (JP)
(74) Representative: Winter, Brandl, Fürniss, Hübner, Röss, Kaiser, Polte - Partnerschaft mbB
(86) International application number: PCT/JP2017/019597
(87) International publication number: WO 2017/208971

(57) **Abstract**

Developed is a technique according to which a nitrogen-containing bicyclic heterocyclic compound can be manufactured directly from nitrogen-containing heterocyclic compounds in a short period of time through a small number of steps without using expensive agents and a starting material that takes time and effort to synthesize. A method for manufacturing a nitrogen-containing bicyclic heterocyclic compound includes a synthesis step of synthesizing a nitrogen-containing bicyclic heterocyclic compound such as bipyrimidine, bipyrazine, or biquinoline by reacting, in a reaction solvent, one nitrogen-containing heterocyclic compound and another nitrogen-containing heterocyclic compound with a solution containing an alkali metal.

## Description

### Technical Field

The present invention relates to a method for manufacturing a nitrogen-containing bicyclic heterocyclic compound constituted by a bipyrimidine compound or the like.

### Background Art

Examples of nitrogen-containing bicyclic heterocyclic compounds include bipyrimidine compounds and bipyrazine compounds, which have a structure in which nitrogen-containing aromatic rings containing two or more nitrogen atoms as hetero atoms are coupled to each other, and biquinoline compounds, which have a structure in which nitrogen-containing heterocycles that are each condensed to a benzene ring are coupled to each other. These compounds are known to be useful as intermediates and raw materials of organic electroluminescent (organic EL) materials, pharmaceuticals, agricultural chemicals, and the like. These compounds are also known to be useful for basic research on the development of organic EL materials, pharmaceuticals, and agricultural chemicals, and a reaction for the reduction of carbon dioxide.

Conventionally, for example, a method for coupling nitrogen-containing heterocyclic compounds in the presence of a nano-cluster catalyst including gold and palladium after halogenating these compounds is reported as a method for manufacturing a nitrogen-containing bicyclic heterocyclic compound (see Patent Document 1). Specifically, Patent Document 1 states that 2,2'-biquinoline can be synthesized at a yield of 97% from 2-chloroquinoline in the presence of the nano-cluster catalyst. Regarding synthesis of a bipyrimidine compound, it is reported that 4,4'-bipyrimidine can be synthesized at a yield of 72% through coupling of 4(3H)-pyrimidinone using a palladium catalyst (palladium (II) acetate) (see Non-Patent Document 1). Regarding manufacturing of a bipyrazine compound, it is reported that 2,2'-bipyrazine can be synthesized at a yield of 76% through coupling of pyrazine using a copper ate complex (LiCu(TMP)₂) and an oxidizing agent (e.g., duroquinone) (see Non-Patent Document 2). In Non-Patent Document 2, it was also confirmed that the yield was 65% in a scale-up experiment, and the yield was significantly low in the absence of the oxidizing agent. Furthermore, it is reported that 2,2'-bipyrazine can be synthesized at a yield of 70% through coupling of 2-iodopyrazine, which is a pyrazine halide, using a palladium catalyst (palladium (II) acetate) (see Non-Patent Document 3).

### Citation List

### Patent Literature

Patent Document 1: JP 2013-184947A

### Non-Patent Literature

Non-Patent Document 1: Abhishek Sharma et al., "Direct Heteroarylation of Tautomerizable Heterocycles into Unsymmetrical and Symmetrical Biheterocycles via Pd/Cu-Catalyzed Phosphonium Coupling", Org. Lett., 2012, 14(7), pp1854-1857
Non-Patent Document 2: Matthew D. Graaf et al., "Photoredox Catalysts: Synthesis of the Bipyrazine Ligand", J. Org. Chem., 2015, 80(3), pp 2032-2035
Non-Patent Document 3: Benjamin J. Coe et al., "Syntheses and electronic and opticalproperties of complexes of the bis(2,2'-bipyrazyl)ruthenium unit", Polyhedron, 2015, 96, pp57-65

### Summary of Invention

### Technical Problem

However, it is necessary to carry out a step of halogenating a nitrogen-containing heterocyclic compound in the methods disclosed in Patent Document 1 and Non-Patent Document 3. Moreover, a very expensive gold catalyst or palladium catalyst is used as a nano-cluster in the method disclosed in Patent Document 1, and a palladium catalyst is also used in the methods disclosed in Non-Patent Document 1 and Non-Patent Document 3, resulting in an increase in cost. In addition, it is necessary to carry out complicated steps for collection, regeneration, and the like of the catalyst in order to use these methods for an industrial application, thus resulting in an increase in the number of steps and complication of the process. In particular, gold and palladium are very rare noble metals, and therefore there is also a problem regarding sustainability. A process for obtaining only 4(3H)-pyrimidinone, which is a starting material used in the method disclosed in Non-Patent Document 1, has not been reported, and there is a problem in that use of this compound is limited due to this compound instead being a by-product produced in synthesis of another compound. LiCu(TMP)₂ used in the method disclosed in Non-Patent Document 2 is thermally unstable and thus decomposes immediately due to a rise in temperature, and therefore, it is necessary to prepare LiCu(TMP)₂ at the time of use and add an oxidizing agent, which causes a problem in that the number of steps increases and the process becomes complicated.

Therefore, there is a demand for the development of a technique according to which a nitrogen-containing bicyclic heterocyclic compound can be manufactured directly from nitrogen-containing heterocyclic compounds in a short period of time through a small number of steps without using expensive agents and a starting material that takes time and effort to synthesize.

### Solution to Problem

As a result of performing intensive studies to solve the foregoing problems, the inventors of the present invention found that a nitrogen-containing bicyclic heterocyclic compound can be synthesized by directly coupling nitrogen-containing heterocyclic compounds to each other without using a gold catalyst or a palladium catalyst, through a reaction between the nitrogen-containing heterocyclic compounds and a solution containing an alkali metal such as a dispersion product obtained by dispersing an alkali metal in a dispersion solvent. Such a synthesis reaction does not require expensive agents such as a gold catalyst and a palladium catalyst and thus is economically advantageous. In addition, with such a synthesis reaction, a nitrogen-containing bicyclic heterocyclic compound can be manufactured in a short period of time through a small number of steps without requiring a complicated chemical technique. The inventors of the present invention achieved the present invention based on these findings.

The present invention is a method for manufacturing a nitrogen-containing bicyclic heterocyclic compound, including a synthesis step of synthesizing a nitrogen-containing bicyclic heterocyclic compound represented by General Formula (III) below by reacting, in a reaction solvent, a nitrogen-containing heterocyclic compound represented by General Formula (I) below and a nitrogen-containing heterocyclic compound represented by General Formula (II) below with a solution containing an alkali metal: 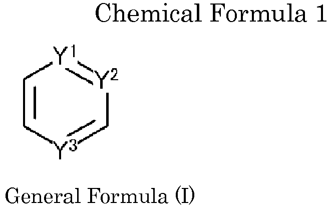 where Y¹, Y², and Y³ in General Formula (I) independently represent a nitrogen atom or a carbon atom, at least two of which represent a nitrogen atom; 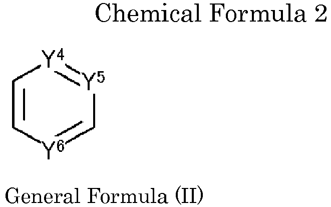 where Y⁴, Y⁵, and Y⁶ in General Formula (II) independently represent a nitrogen atom or a carbon atom, at least one of which represents a nitrogen atom; and 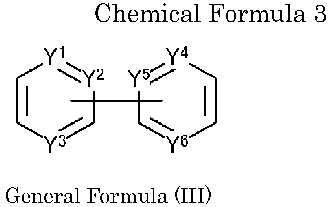 where Y¹, Y², and Y³ in General Formula (III) independently represent a nitrogen atom or a carbon atom, at least two of which represent a nitrogen atom, and are respectively the same as Y¹, Y², and Y³ in General Formula (I), while Y⁴, Y⁵, and Y⁶ in General Formula (III) independently represent a nitrogen atom or a carbon atom, at least one of which represents a nitrogen atom, and are respectively the same as Y⁴, Y⁵, and Y⁶ in General Formula (II).

Conventionally, metallic catalysts such as gold and palladium and reducing agents such as lithium have been widely used in syntheses of nitrogen-containing bicyclic heterocyclic compounds such as bipyrimidine compounds, bipyrazine compounds, and biquinoline compounds. However, catalysts made of gold, palladium, and the like are very expensive, thus resulting in an increase in cost. In addition, it is necessary to carry out steps for collection, regeneration, and the like of the catalyst in order to use these catalysts for an industrial application, which causes a problem in that the number of steps increases and the process becomes complicated.

With this method, a solution containing an alkali metal, which is easy to handle, such as a dispersion product obtained by dispersing an alkali metal in a dispersion solvent is used, and therefore, a catalyst made of gold, palladium, or the like need not be used. Accordingly, it is possible to reduce the cost, and synthesize a nitrogen-containing bicyclic heterocyclic compound by directly coupling nitrogen-containing heterocyclic compounds to each other in a short period of time through a small number of steps without requiring a complicated chemical technique. In addition, a nitrogen-containing bicyclic heterocyclic compound can be synthesized with significant economic and industrial advantages. In particular, alkali metals as typified by sodium are very widely distributed over the earth, and therefore, this technique is excellent from the viewpoint of sustainability.

In another aspect of the manufacturing method, the Y¹ represents a carbon atom, the Y² and the Y³ represent a nitrogen atom, the Y⁴ represents a carbon atom, and the Y⁵ and the Y⁶ represent a nitrogen atom.

With this method, a bipyrimidine compound can be manufactured through direct coupling of pyrimidine. As a result, a bipyrimidine compound such as 4,4'-bipyrimidine that can be favorably used for manufacturing of organic EL materials, pharmaceuticals, agricultural chemicals, dyes, and the like, basic research thereon, and a reaction for the reduction of carbon dioxide can be produced in a simple manner in a short period of time at low cost.

In another aspect of the manufacturing method, the Y¹ and the Y³ represent a nitrogen atom, the Y² represents a carbon atom, the Y⁴ and the Y⁶ represent a nitrogen atom, and the Y⁵ represents a carbon atom.

With this method, a bipyrazine compound can be manufactured through direct coupling of pyrazine. As a result, a bipyrazine compound such as 2,2'-bipyrazine that can be favorably used for manufacturing of organic EL materials, pharmaceuticals, agricultural chemicals, dyes, and the like, basic research thereon, and a reaction for the reduction of carbon dioxide can be produced in a simple manner in a short period of time at low cost.

Furthermore, the present invention is a method for manufacturing a nitrogen-containing bicyclic heterocyclic compound, including a synthesis step of synthesizing a nitrogen-containing bicyclic heterocyclic compound represented by General Formula (VI) below by reacting, in a reaction solvent, a nitrogen-containing heterocyclic compound represented by General Formula (IV) below and a nitrogen-containing heterocyclic compound represented by General Formula (V) below with a solution containing an alkali metal: 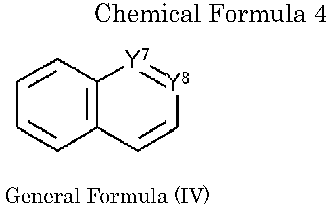 where Y⁷ and Y⁸ in General Formula (IV) independently represent a nitrogen atom or a carbon atom, one of which represents a nitrogen atom and the other of which represents a carbon atom; 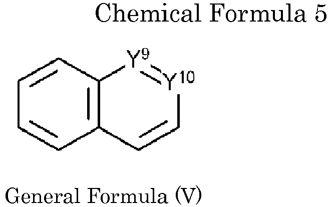 where Y⁹ and Y¹⁰ in General Formula (V) independently represent a nitrogen atom or a carbon atom, one of which represents a nitrogen atom and the other of which represents a carbon atom; and 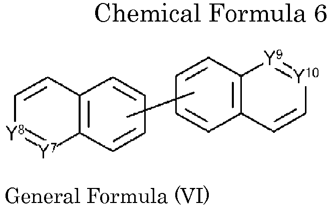 where Y⁷ and Y⁸ in General Formula (VI) independently represent a nitrogen atom or a carbon atom, one of which represents a nitrogen atom and the other of which represents a carbon atom, and are respectively the same as Y⁷ and Y⁸ in General Formula (IV), while Y⁹ and Y¹⁰ in General Formula (VI) independently represent a nitrogen atom or a carbon atom, one of which represents a nitrogen atom and the other of which represents a carbon atom, and are respectively the same as Y⁹ and Y¹⁰ in General Formula (V).

With this method, a biquinoline compound can be manufactured through direct coupling of quinoline. As a result, a biquinoline compound that can be favorably used for manufacturing of organic EL materials, pharmaceuticals, agricultural chemicals, dyes, and the like, basic research thereon, and a reaction for the reduction of carbon dioxide can be produced in a simple manner in a short period of time at low cost.

In another aspect of the manufacturing method, the solution containing an alkali metal is a dispersion product obtained by dispersing the alkali metal in a dispersion solvent, and the method includes: a deactivation step of deactivating the alkali metal remaining in the reaction solution containing the synthesized nitrogen-containing bicyclic heterocyclic compound; a first dissolution step of dissolving the dispersion solvent by adding a normal paraffin-based organic solvent to the reaction solution in which the alkali metal has been deactivated; and a separation step of separating an organic layer containing the normal paraffin-based organic solvent and an aqueous layer containing the nitrogen-containing bicyclic heterocyclic compound.

With this method, a dispersion solvent such as insulating oil contained in the reaction solution in which the alkali metal has been deactivated is dissolved in a normal paraffin-based organic solvent in the first dissolution step and then isolated from the aqueous layer containing the nitrogen-containing bicyclic heterocyclic compound in the separation step. As a result, the insulation oil does not attach to the nitrogen-containing bicyclic heterocyclic compound, and the purity of the nitrogen-containing bicyclic heterocyclic compound can thus be improved.

Another aspect of the manufacturing method further includes: a second dissolution step of dissolving the nitrogen-containing bicyclic heterocyclic compound in a low boiling point solvent having a boiling point lower than the melting point of the nitrogen-containing bicyclic heterocyclic compound by adding the low boiling point solvent to the aqueous layer; and a precipitation step of precipitating the nitrogen-containing bicyclic heterocyclic compound by evaporating the low boiling point solvent.

With this method, the nitrogen-containing bicyclic heterocyclic compound can be collected in the form of a solid merely by evaporating the low boiling point solvent. Therefore, this method requires less manufacturing man-hours and is thus performed efficiently.

### Brief Description of Drawings

FIG. 1 is a diagram illustrating a mechanism of a reaction for synthesis of a nitrogen-containing bicyclic heterocyclic compound (4,4'-bipyrimidine).
FIG. 2 is a diagram illustrating a mechanism of a reaction for synthesis of a nitrogen-containing bicyclic heterocyclic compound (2,2'-bipyrazine).
FIG. 3 is a flowchart illustrating a method for manufacturing a nitrogen-containing bicyclic heterocyclic compound.
FIG. 4 is a diagram summarizing the synthesis conditions and the synthesis results in examples in which the synthesis of a nitrogen-containing bicyclic heterocyclic compound was investigated.

### Description of Embodiments

Hereinafter, methods for manufacturing a nitrogen-containing bicyclic heterocyclic compound according to embodiments of the present invention will be described in detail. However, the present invention is not limited to the embodiments described below.

### 1. Synthesis of nitrogen-containing bicyclic heterocyclic compound

### 1-1. Synthesis of nitrogen-containing bicyclic heterocyclic compound including a heterocyclic structure containing two or more nitrogen atoms as hetero atoms

With a method for manufacturing a nitrogen-containing bicyclic heterocyclic compound according to an embodiment of the present invention, a nitrogen-containing bicyclic heterocyclic compound represented by General Formula (III) below is synthesized by reacting, in a reaction solvent, a nitrogen-containing heterocyclic compound represented by General Formula (I) below and a nitrogen-containing heterocyclic compound represented by General Formula (II) below with a solution containing an alkali metal. The "nitrogen-containing bicyclic heterocyclic compound" as used herein refers to a compound having a structure in which two nitrogen-containing heterocycles are linked to each other in its molecule, and also encompasses a compound having a structure in which a cyclic structure other than a nitrogen-containing heterocycle is further linked to such a compound. 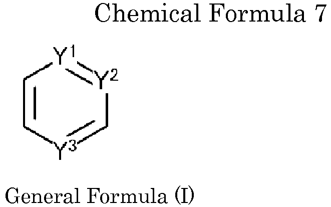 (Here, Y¹, Y², and Y³ in General Formula (I) independently represent a nitrogen atom or a carbon atom, at least two of which represent a nitrogen atom.) 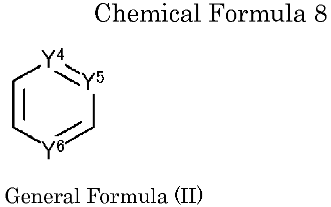 (Here, Y⁴, Y⁵, and Y⁶ in General Formula (II) independently represent a nitrogen atom or a carbon atom, at least one of which represents a nitrogen atom.) 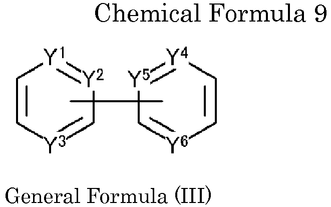 (Here, Y¹, Y², and Y³ in General Formula (III) independently represent a nitrogen atom or a carbon atom, at least two of which represent a nitrogen atom, and are respectively the same as Y¹, Y², and Y³ in General Formula (I), while Y⁴, Y⁵, and Y⁶ in General Formula (III) independently represent a nitrogen atom or a carbon atom, at least one of which represents a nitrogen atom, and are respectively the same as Y⁴, Y⁵, and Y⁶ in General Formula (II).)

The nitrogen-containing heterocyclic compound represented by General Formula (I), which is one of the starting materials used in the method for manufacturing a nitrogen-containing bicyclic heterocyclic compound according to this embodiment, has, in its ring structure, a heterocyclic structure containing a nitrogen atom as a hetero atom. The "heterocyclic structure" as used herein refers to a structure that contains not only a carbon atom but also an atom (hetero atom) other than a carbon atom as ring forming atoms and in which these atoms are linked to form an annular shape. The nitrogen-containing heterocyclic compound represented by General Formula (I) has a heterocyclic structure containing two or more nitrogen atoms as hetero atoms other than carbon atoms. It is preferable that a six-membered ring structure in which six atoms are linked to form an annular shape is included, and the six atoms are constituted by four carbon atoms and two nitrogen atoms. At this time, there is no particular limitation on the positions of the nitrogen atoms. Accordingly, examples of the nitrogen-containing heterocyclic compound represented by General Formula (I) include a compound having a pyridazine ring in which two nitrogen atoms are located at ortho positions (Y¹ and Y² represent a nitrogen atom, and Y³ represents a carbon atom), a compound having a pyrimidine ring in which two nitrogen atoms are located at meta positions (Y¹ represents a carbon atom, and Y² and Y³ represent a nitrogen atom), and a compound having a pyrazine ring in which two nitrogen atoms are located at para positions (Y¹ and Y³ represent a nitrogen atom, and Y² represents a carbon atom).

Furthermore, in the nitrogen-containing heterocyclic compound represented by General Formula (I), a hydrogen atom located at any position of the nitrogen-containing heterocycle is optionally substituted by a substituent. There is no particular limitation on whether or not a substituent is present, and in a case where a substituent is present, there are no particular limitations on the position, number, and type of the substituent. These are set as appropriate depending on a target compound, namely the nitrogen-containing bicyclic heterocyclic compound represented by General Formula (III).

An example of the substituent is a hydrocarbon group. There is no particular limitation on the hydrocarbon group as long as it contains preferably 1 to 10 carbon atoms and particularly preferably 1 to 5 carbon atoms. The hydrocarbon group is preferably a saturated linear or branched alkyl group, and specific examples thereof include a methyl group, an ethyl group, a propyl group, an isopropyl group, an n-butyl group, an isobutyl group, an s-butyl group, a t-butyl group, an n-pentyl group, an isopentyl group, a neopentyl group, a t-pentyl group, an s-pentyl group, a 2-methylbutyl group, a 1-ethylpropyl group, a 2-ethylpropyl group, an n-hexyl group, an isohexyl group, a neohexyl group, a t-hexyl group, a 2,2-dimethylbutyl group, a 2-methylpentyl group, a 3-methylpentyl group, a 1-ethylbutyl group, a 2-ethylbutyl group, a 1-propylpropyl group, an n-heptyl group, an isoheptyl group, an s-heptyl group, a t-heptyl group, a 2,2-dimethylpentyl group, a 3,3-dimethylpentyl group, a 1-methylhexyl group, a 2-methylhexyl group, a 3-methylhexyl group, a 4-methylhexyl group, a 1-ethylpentyl group, a 2-ethylpentyl group, a 3-ethylpentyl group, a 1-propylbutyl group, a 2-propylbutyl group, an n-octyl group, an isooctyl group, a t-octyl group, a neooctyl group, a 2,2-dimethylhexyl group, a 3,3-dimethylhexyl group, a 4,4-dimethylhexyl group, a 1-methylheptyl group, a 2-methylheptyl group, a 3-methylheptyl group, a 4-methylheptyl group, a 5-methylheptyl group, a 1-ethylhexyl group, a 2-ethylhexyl group, a 3-ethylhexyl group, a 4-ethylhexyl group, a 1-propylpentyl group, a 2-propylpentyl group, a 3-propylpentyl group, an n-nonyl group, an isononyl group, a t-nonyl group, a 1-methyloctyl group, a 2-methyloctyl group, a 3-methyloctyl group, a 4-methyloctyl group, a 5-methyloctyl group, a 6-methyloctyl group, an n-decyl group, an isodecyl group, a t-decyl group, a 1-methylnonyl group, a 2-methylnonyl group, a 3-methylnonyl group, a 4-methylnonyl group, a 5-methylnonyl group, a 6-methylnonyl group, and a 7-methylnonyl group.

Specific examples of the nitrogen-containing heterocyclic compound represented by General Formula (I) include pyridazine, pyrimidine, and pyrazine, which are six-membered nitrogen-containing heterocyclic compounds having two nitrogen atoms in their ring structure and in which hydrogen atoms linked to the carbon atoms included in the ring structure are not substituted by substituents.

A commercially available compound or a compound manufactured using a method known in the art may be used as the nitrogen-containing heterocyclic compound represented by General Formula (I).

The nitrogen-containing heterocyclic compound represented by General Formula (II), which is the other of the starting materials used in the method for manufacturing a nitrogen-containing bicyclic heterocyclic compound according to this embodiment, has, in its ring structure, a heterocyclic structure containing a nitrogen atom as a hetero atom. The nitrogen-containing heterocyclic compound represented by General Formula (II) has a heterocyclic structure containing one or more nitrogen atoms as hetero atoms other than carbon atoms. It is preferable that a six-membered ring structure in which six atoms are linked to form an annular shape is included, and the six atoms are constituted by four carbon atoms and two nitrogen atoms or five carbon atoms and one nitrogen atom. At this time, there is no particular limitation on the positions of the nitrogen atoms. Accordingly, examples of the nitrogen-containing heterocyclic compound represented by General Formula (II) include a compound having a pyridazine ring in which two nitrogen atoms are located at ortho positions (Y⁴ and Y⁵ represent a nitrogen atom, and Y⁶ represents a carbon atom), a compound having a pyrimidine ring in which two nitrogen atoms are located at meta positions (Y⁴ represents a carbon atom, and Y⁵ and Y⁶ represent a nitrogen atom), a compound having a pyrazine ring in which two nitrogen atoms are located at para positions (Y⁴ and Y⁶ represent a nitrogen atom, and Y⁵ represents a carbon atom), and a compound having a pyridine ring in which one nitrogen atom is located at any position (one of Y⁴, Y⁵, and Y⁶ represents a nitrogen atom, and the other two represent a carbon atom).

Furthermore, in the nitrogen-containing heterocyclic compound represented by General Formula (II), a hydrogen atom located at any position of the nitrogen-containing heterocycle is optionally substituted by a substituent. There is no particular limitation on whether or not a substituent is present, and in a case where a substituent is present, there are no particular limitations on the position, number, and type of the substituent. These are set as appropriate depending on a target compound, namely a nitrogen-containing bicyclic heterocyclic compound.

An example of the substituent is a hydrocarbon group. There is no particular limitation on the hydrocarbon group as long as it contains preferably 1 to 10 carbon atoms and particularly preferably 1 to 5 carbon atoms. Specific examples of the hydrocarbon group include those described above.

Specific examples of the nitrogen-containing heterocyclic compound represented by General Formula (II) include pyridazine, pyrimidine, and pyrazine, which are six-membered nitrogen-containing heterocyclic compounds having two nitrogen atoms in their ring structure and in which hydrogen atoms linked to the carbon atoms included in the ring structure are not substituted by substituents, as well as pyridine, which is a six-membered nitrogen-containing heterocyclic compound having one nitrogen atom in its ring structure and in which hydrogen atoms linked to the carbon atoms included in the ring structure are not substituted by substituents.

A commercially available compound or a compound manufactured using a method known in the art may be used as the nitrogen-containing heterocyclic compound represented by General Formula (II).

It should be noted that the nitrogen-containing heterocyclic compound represented by General Formula (I) and the nitrogen-containing heterocyclic compound represented by General Formula (II) may be the same or different as long as they have a nitrogen-containing heterocycle. Therefore, the method for manufacturing a nitrogen-containing bicyclic heterocyclic compound according to this embodiment encompasses both a method based on homo-coupling with which two molecules having the same structure are linked to form one molecule and a method based on cross-coupling with which two molecules having different structures are linked to form one molecule.

A target compound obtained using the method for manufacturing a nitrogen-containing bicyclic heterocyclic compound according to this embodiment is the nitrogen-containing bicyclic heterocyclic compound represented by General Formula (III). In the nitrogen-containing bicyclic heterocyclic compound represented by General Formula (III), two nitrogen-containing heterocycles are linked to each other, and a carbon atom located at any position of one of the nitrogen-containing heterocycles and a carbon atom located at any position of the other of the nitrogen-containing heterocycles are linked to each other.

Examples of the nitrogen-containing bicyclic heterocyclic compound represented by General Formula (III) include a bipyrimidine compound having a structure in which any of 2-positions, 4-positions, and 5-positions of pyrimidine rings having two nitrogen atoms at meta positions are linked to each other, and a bipyrazine compound having a structure in which 2-positions of pyrazine rings having two nitrogen atoms at para positions are linked to each other. Furthermore, examples thereof also include a compound having a structure in which either of 3-position or 4-position of a pyridazine ring and any of 2- to 6-positions of a pyridine ring are linked to each other, a compound having a structure in which any of 2-position, 4-position, and 5-position of a pyrimidine ring and any of 2- to 6-positions of a pyridine ring are linked to each other, a compound having a structure in which 2-position of a pyrazine ring and any of 2- to 6-positions of a pyridine ring are linked to each other, a compound having a structure in which either of 3-position or 4-position of a pyridazine ring and any of 2-position, 4-position, and 5-position of a pyrimidine ring are linked to each other, a compound having a structure in which either of 3-position or 4-position of a pyridazine ring and 2-position of a pyrazine ring are linked to each other, and a compound having a structure in which any of 2-position, 4-position, and 5-position of a pyrimidine ring and 2-position of a pyrazine ring are linked to each other. It should be noted that the positions in the above-described ring structures are numbered such that the positional number for a nitrogen atom, which is a hetero atom, is the smallest.

Furthermore, in the nitrogen-containing bicyclic heterocyclic compound represented by General Formula (III), a hydrogen atom located at any position of the coupled nitrogen-containing heterocycles is optionally substituted by a substituent. In a case where a substituent is present, only one of the nitrogen-containing heterocycles has a substituent, or both the nitrogen-containing heterocycles have a substituent. Although there are no particular limitations on the positions and numbers of the substituent, the substituent is derived from the substituent for the nitrogen-containing heterocyclic compound represented by General Formula (I) above and the nitrogen-containing heterocyclic compound represented by General Formula (II) above, which are the starting materials.

Therefore, an example of the substituent is a hydrocarbon group. There is no particular limitation on the hydrocarbon group as long as it contains preferably 1 to 10 carbon atoms and particularly preferably 1 to 5 carbon atoms. Specific examples of the hydrocarbon group include those described above.

Specific examples of the nitrogen-containing bicyclic heterocyclic compound represented by General Formula (III) include 2,2'-bipyrimidine, 2,4'-bipyrimidine, 2,5'-bipyrimidine, 4,4'-bipyrimidine, 4,5'-bipyrimidine, 5,5'-bipyrimidine, and 2,2'-bipyrazine. Furthermore, specific examples thereof also include 3-(2-pyridyl)pyridazine, 3-(3-pyridyl)pyridazine, 3-(4-pyridyl)pyridazine, 3-(5-pyridyl)pyridazine, 3-(6-pyridyl)pyridazine, 4-(2-pyridyl)pyridazine, 4-(3-pyridyl)pyridazine, 4-(4-pyridyl)pyridazine, 4-(5-pyridyl)pyridazine, 4-(6-pyridyl)pyridazine, 2-(2-pyridyl)pyrimidine, 2-(3-pyridyl)pyrimidine, 2-(4-pyridyl)pyrimidine, 2-(5-pyridyl)pyrimidine, 2-(6-pyridyl)pyrimidine, 4-(2-pyridyl)pyrimidine, 4-(3-pyridyl)pyrimidine, 4-(4-pyridyl)pyrimidine, 4-(5-pyridyl)pyrimidine, 4-(6-pyridyl)pyrimidine, 5-(2-pyridyl)pyrimidine, 5 - (3-pyridyl)pyrimidine, 5 - (4-pyridyl)pyrimidine, 5-(5-pyridyl)pyrimidine, 5-(6-pyridyl)pyrimidine, 2-(2-pyridyl)pyrazine, 2-(3-pyridyl)pyrazine, 2-(4-pyridyl)pyrazine, 2-(5-pyridyl)pyrazine, 2-(6-pyridyl)pyrazine, 3-(2-pyrimidyl)pyridazine, 3-(4-pyrimidyl)pyridazine, 3-(5-pyrimidyl)pyridazine, 4-(2-pyrimidyl)pyridazine, 4-(4-pyrimidyl)pyridazine, 4-(5-pyrimidyl)pyridazine, 3-(2-pyrazyl)pyridazine, 4-(2-pyrazyl)pyridazine, 2-(2-pyrazyl)pyrimidine, 4-(2-pyrazyl)pyrimidine, and 5-(2-pyrazyl)pyrimidine.

Examples of a solution containing an alkali metal used in the method for manufacturing a nitrogen-containing bicyclic heterocyclic compound according to this embodiment include a dispersion product obtained by dispersing an alkali metal in a dispersion solvent, and a melt of an alkali metal. The dispersion product obtained by dispersing an alkali metal in a dispersion solvent is a dispersion product obtained by dispersing minute particles of an alkali metal in an insoluble solvent, or a dispersion product obtained by dispersing an alkali metal in a liquid form in an insoluble solvent,. Examples of the alkali metal include sodium, potassium, lithium and alloys thereof. The average particle diameter of the minute particles is preferably less than 10 µm, and the minute particles having an average particle diameter of less than 5 µm can be used particularly preferably. The diameter of a sphere having a projected area equal to the projected area obtained through image analyses of photomicrographs is taken as the average particle diameter.

A solvent known in the art can be used as the dispersion solvent as long as minute particles of an alkali metal or an alkali metal in a liquid form can be dispersed in an insoluble solvent, and the reaction of a coupling target compound with the dispersion product of the alkali metal is not inhibited. Examples thereof include aromatic solvents such as xylene and toluene, normal paraffin-based solvents such as decane, heterocyclic compound-based solvents such as tetrahydrothiophene, and mixed solvents thereof.

In the method for manufacturing a nitrogen-containing bicyclic heterocyclic compound according to this embodiment, in addition to the dispersion product obtained by dispersing an alkali metal in a dispersion solvent, a melt obtained by melting an alkali metal can also be used in the same manner. A melt can be obtained by heating an alkali metal to its melting temperature using a means known in the art.

Hereinafter, the dispersion product obtained by dispersing an alkali metal in a dispersion solvent and a melt obtained by melting an alkali metal may be abbreviated as "SD". SD is an abbreviation of "sodium dispersion". In examples described below, a dispersion product in which sodium is dispersed as the alkali metal is used, and therefore, the dispersion product is denoted by the abbreviation "SD". However, alkali metals other than sodium are also encompassed by the abbreviation "SD", and the abbreviation "SD" is construed as encompassing not only a dispersion product but also a melt.

A solvent known in the art can be used as the reaction solvent used in the method for manufacturing a nitrogen-containing bicyclic heterocyclic compound according to this embodiment as long as the reaction of the starting materials with SD is not inhibited. Examples thereof include ether-based solvents, normal paraffin-based solvents, aromatic solvents, amine-based solvents, and heterocyclic compound-based solvents. As the ether-based solvent, a cyclic ether-based solvent is preferable. Tetrahydrofuran (which may be abbreviated as "THF" hereinafter), which is an inexpensive solvent to be used for many purposes in the art, is particularly preferable. THF has excellent substance dissolvability and is thus advantageous in that the efficiency of contact between the starting materials and SD can be improved, the efficiency of manufacturing a nitrogen-containing bicyclic heterocyclic compound can be improved, and a highly pure nitrogen-containing bicyclic heterocyclic compound can be obtained. As the normal paraffin-based solvent, normal decane or the like is particularly preferable. As the aromatic solvent, xylene, toluene, benzene, or the like is preferable, and a halogenated aromatic solvent such as chlorobenzene or fluorobenzene can be used. As the amine-based solvent, ethylenediamine or the like can be favorably used. As the heterocyclic compound-based solvent, tetrahydrothiophene or the like can be used. These solvents may be used alone or in combination of two or more as a mixed solvent. Here, the above-described dispersion solvent and the reaction solvent may be the same or different.

In the method for manufacturing a nitrogen-containing bicyclic heterocyclic compound according to this embodiment, the nitrogen-containing heterocyclic compound represented by General Formula (I) and the nitrogen-containing heterocyclic compound represented by General Formula (II) are used as starting materials (also referred to as "monomers" hereinafter), and the monomers are reacted with SD in the reaction solvent to manufacture the nitrogen-containing bicyclic heterocyclic compound represented by General Formula (III). Hereinafter, reaction conditions will be described in detail.

There is no particular limitation on the reaction temperature, and the reaction temperature can be set as appropriate depending on the types and amounts of the monomers, SD, and the reaction solvent, the reaction pressure, and the like. Specifically, it is preferable to set the reaction temperature to a temperature lower than the boiling point of the reaction solvent. Under increased pressure, a boiling point is higher than that under atmospheric pressure, and the reaction temperature can thus be set to a higher temperature. The reaction can also be performed at room temperature, and the reaction temperature is preferably 0 to 100°C, more preferably 20 to 80°C, and even more preferably room temperature to 50°C. Although it is not necessary to provide a particular temperature controlling means for heating, cooling, and the like, a temperature controlling means may be provided as necessary.

There is also no particular limitation on the reaction time, and it is sufficient that the reaction time is set as appropriate depending on the types and amounts of the monomers, SD, and the reaction solvent, the reaction pressure, the reaction temperature, and the like. In general, the reaction time is 1 to 24 hours and preferably 1 to 6 hours.

Moreover, all of the agents required in the method for manufacturing a nitrogen-containing bicyclic heterocyclic compound according to this embodiment, namely the monomers, SD, the reaction solvent, and the like, can be handled stably in an atmosphere, thus making it possible to perform the reaction under normal pressure conditions in an atmosphere. However, the reaction may also be performed in an inert gas atmosphere that is filled with argon gas, nitrogen gas, or the like, and there is no particular limitation.

The usage amount of SD can be set as appropriate depending on the types and amounts of the monomers and the reaction solvent. When the monomers, namely the nitrogen-containing heterocyclic compound represented by General Formula (I) and the nitrogen-containing heterocyclic compound represented by General Formula (II), are reacted with SD, it is preferable that 1 to 4 ml of the reaction solvent is used with respect to 1 mmol of the total substance amount of the monomers, and SD is reacted with the monomers in the reaction solvent under conditions where the ratio of the amount of the monomers to the amount of SD is 1:1 to 1:3. When different types of monomers are used, it is preferable to prepare the nitrogen-containing heterocyclic compound represented by General Formula (I), the nitrogen-containing heterocyclic compound represented by General Formula (II), and SD such that a molar equivalent ratio therebetween is 1:1:1 to 1:1:3. The substance amount of SD herein means the substance amount in terms of the alkali metal contained in SD.

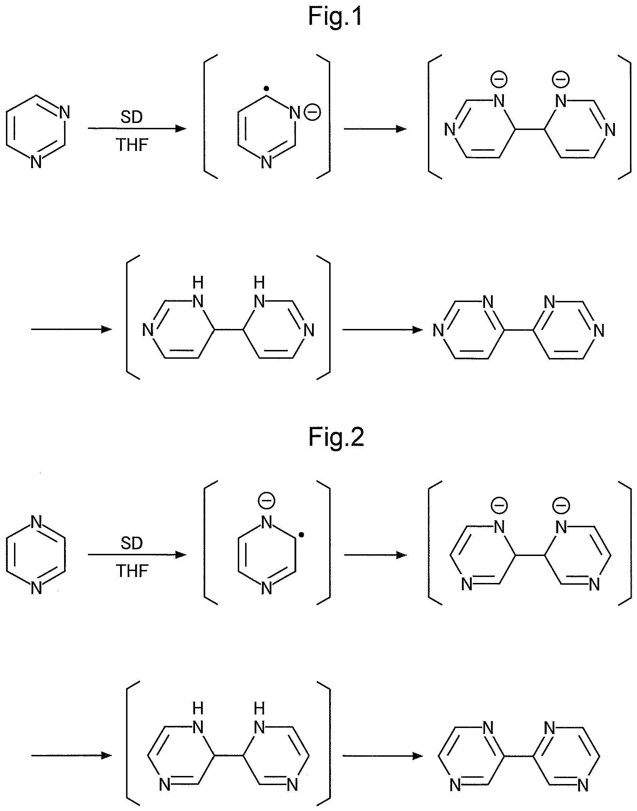

In the method for manufacturing a nitrogen-containing bicyclic heterocyclic compound according to this embodiment, the mechanism of the reaction for the synthesis of the nitrogen-containing bicyclic heterocyclic compound represented by General Formula (III) is as follows. When the nitrogen-containing heterocyclic compound represented by General Formula (I) and the nitrogen-containing heterocyclic compound represented by General Formula (II), which are the starting materials, are reacted with SD in the reaction solvent, an electron is released from the sodium metal included in SD. The electron released from the sodium metal enters the nitrogen-containing heterocycle, and a radical anion is thus generated. The nitrogen-containing heterocycles are linked to each other through a radical anion coupling reaction, and nitrogen atoms adjacent to the linking site receive hydrogen atoms. It is assumed that the thus obtained compound is oxidized, and the nitrogen-containing bicyclic heterocyclic compound represented by General Formula (III) is thus obtained. Here, FIGS. 1 and 2 show, as examples, diagrams that respectively illustrate mechanisms of a reaction for the synthesis of 4,4'-bipyrimidine and a reaction for the synthesis of 2,2'-bipyrazine that are investigated in Examples 1 and 2 described below.

With the method for manufacturing a nitrogen-containing bicyclic heterocyclic compound according to this embodiment, using SD in the reaction for coupling the nitrogen-containing heterocyclic compounds, which are starting materials, makes it possible to cause the reaction to progress stably and efficiently even when the molar equivalent ratio of SD to the starting materials is small. Moreover, the reaction is performed using a simple process including a single step. Accordingly, the reaction time can be shortened, and the usage amounts of the reaction agents can be suppressed to a minimum level, thus making it possible to reduce cost in particular.

### 1-2. Synthesis of nitrogen-containing bicyclic heterocyclic compound including quinoline ring or isoquinoline ring according to another embodiment of the present invention

With a method for manufacturing a nitrogen-containing bicyclic heterocyclic compound according to another embodiment of the present invention, a nitrogen-containing bicyclic heterocyclic compound represented by General Formula (VI) below is synthesized by reacting, in a reaction solvent, a nitrogen-containing heterocyclic compound represented by General Formula (IV) below and a nitrogen-containing heterocyclic compound represented by General Formula (V) below with a solution containing an alkali metal. 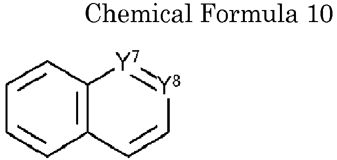 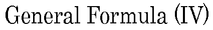 (Here, Y⁷ and Y⁸ in General Formula (IV) independently represent a nitrogen atom or a carbon atom, one of which represents a nitrogen atom and the other of which represents a carbon atom.) 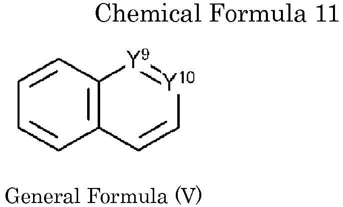 (Here, Y⁹ and Y¹⁰ in General Formula (V) independently represent a nitrogen atom or a carbon atom, one of which represents a nitrogen atom and the other of which represents a carbon atom.) 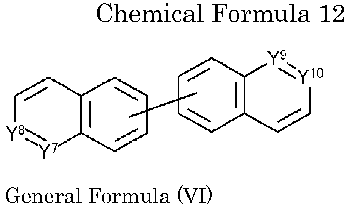 (Here, Y⁷ and Y⁸ in General Formula (VI) independently represent a nitrogen atom or a carbon atom, one of which represents a nitrogen atom and the other of which represents a carbon atom, and are respectively the same as Y⁷ and Y⁸ in General Formula (IV), while Y⁹ and Y¹⁰ in General Formula (VI) independently represent a nitrogen atom or a carbon atom, one of which represents a nitrogen atom and the other of which represents a carbon atom, and are respectively the same as Y⁹ and Y¹⁰ in General Formula (V).)

The nitrogen-containing heterocyclic compound represented by General Formula (IV), which is one of the starting materials used in the method for manufacturing a nitrogen-containing bicyclic heterocyclic compound according to this embodiment, has a quinoline ring structure (Y⁷ represents a nitrogen atom and Y⁸ represents a carbon atom) or an isoquinoline ring structure (Y⁷ represents a carbon atom and Y⁸ represents a nitrogen atom) in which a benzene ring is condensed to a pyridine ring, which is constituted by five carbon atoms and one nitrogen atom that are linked to form an annular shape. It should be noted that the quinoline ring and the isoquinoline ring may be collectively referred to as "quinoline ring system" hereinafter.

Furthermore, in the nitrogen-containing heterocyclic compound represented by General Formula (IV), a substituent may be substituted for a hydrogen atom located at any position of the quinoline ring system. There is no particular limitation on whether or not a substituent is present, and in a case where a substituent is present, there are no particular limitations on the position, number, and type of the substituent. These are set as appropriate depending on a target compound, namely a nitrogen-containing bicyclic heterocyclic compound.

An example of the substituent is a hydrocarbon group. There is no particular limitation on the hydrocarbon group as long as it contains preferably 1 to 10 carbon atoms and particularly preferably 1 to 5 carbon atoms. Specific examples of the hydrocarbon group include those described in item 1-1.

A specific example of the nitrogen-containing heterocyclic compound represented by General Formula (IV) is quinoline, which is a nitrogen-containing heterocyclic compound having a quinoline ring and in which hydrogen atoms linked to the carbon atoms included in the quinoline ring are not substituted by substituents.

A commercially available compound or a compound manufactured using a method known in the art may be used as the nitrogen-containing heterocyclic compound represented by General Formula (IV).

Similarly to the nitrogen-containing heterocyclic compound represented by General Formula (IV), the nitrogen-containing heterocyclic compound represented by General Formula (V), which is the other of the starting materials used in the method for manufacturing a nitrogen-containing bicyclic heterocyclic compound according to this embodiment, has a quinoline ring structure (Y⁹ represents a nitrogen atom and Y¹⁰ represents a carbon atom) or an isoquinoline ring structure (Y⁹ represents a carbon atom and Y¹⁰ represents a nitrogen atom) in which a benzene ring is condensed to a pyridine ring, which is constituted by five carbon atoms and one nitrogen atom that are linked to form an annular shape.

Furthermore, in the nitrogen-containing heterocyclic compound represented by General Formula (V), a substituent may be substituted for a hydrogen atom located at any position of the quinoline ring system. There is no particular limitation on whether or not a substituent is present, and in a case where a substituent is present, there are no particular limitations on the position, number, and type of the substituent. These are set as appropriate depending on a target compound, namely a nitrogen-containing bicyclic heterocyclic compound.

An example of the substituent is a hydrocarbon group. There is no particular limitation on the hydrocarbon group as long as it contains preferably 1 to 10 carbon atoms and particularly preferably 1 to 5 carbon atoms. Specific examples of the hydrocarbon group include those described in item 1-1.

A specific example of the nitrogen-containing heterocyclic compound represented by General Formula (V) is quinoline, which is a nitrogen-containing heterocyclic compound having a quinoline ring and in which hydrogen atoms linked to the carbon atoms included in the quinoline ring are not substituted by substituents.

A commercially available compound or a compound manufactured using a method known in the art may be used as the nitrogen-containing heterocyclic compound represented by General Formula (V).

It should be noted that the nitrogen-containing heterocyclic compound represented by General Formula (IV) and nitrogen-containing heterocyclic compound represented by General Formula (V) have a common point in that they have a quinoline ring-based structure, but may be the same or different in that a substituent is present or absent, and in a case where a substituent is present, they may be the same or different regarding the number, type, and position of the substituent.

A target compound obtained using the method for manufacturing a nitrogen-containing bicyclic heterocyclic compound according to this embodiment is the nitrogen-containing bicyclic heterocyclic compound represented by General Formula (VI). In the nitrogen-containing bicyclic heterocyclic compound represented by General Formula (VI), two quinoline ring systems are linked to each other, and a carbon atom located at any position of one of the quinoline ring systems and a carbon atom located at any position of the other of the quinoline ring systems are linked to each other. Linking quinoline ring systems encompasses linking quinoline rings, linking isoquinoline rings, and linking a quinoline ring and an isoquinoline ring. The linking site is any of 2- to 4-positions in the case of a quinoline ring and any of 1-, 3- and 4-positions in the case of an isoquinoline ring. It should be noted that, regarding the positional numbers in the above-described structures, the position of a nitrogen atom on a side where a nitrogen atom whose positional number is the smallest is located, out of the atoms adjacent to the condensed atoms among the ring forming atoms in the quinoline ring system, is numbered as 1-position.

Furthermore, in the nitrogen-containing bicyclic heterocyclic compound represented by General Formula (VI), a hydrogen atom located at any position of the two quinoline rings is optionally substituted by a substituent. In a case where a substituent is present, only one of the quinoline ring systems has a substituent, or both the quinoline ring systems have a substituent. Although there are no particular limitations on the positions and numbers of the substituent, the substituent is derived from the substituent for the nitrogen-containing heterocyclic compound represented by General Formula (IV) and the nitrogen-containing heterocyclic compound represented by General Formula (V), which are the starting materials.

Therefore, an example of the substituent is a hydrocarbon group. There is no particular limitation on the hydrocarbon group as long as it contains preferably 1 to 10 carbon atoms and particularly preferably 1 to 5 carbon atoms. Specific examples of the hydrocarbon group include those described in item 1-1 above.

Specific examples of the nitrogen-containing bicyclic heterocyclic compound represented by General Formula (VI) include 2,2-biquinoline, 2,3-biquinoline, 2,4-biquinoline, 3,3-biquinoline, 3,4-biquinoline, 4,4-biquinoline, 1,1-biisoquinoline, 1,3-biisoquinoline, 1,4-biisoquinoline, 3,3-biisoquinoline, 3,4-biisoquinoline, 4,4-biisoquinoline, 2-(1-isoquinolyl)quinoline, 2-(3-isoquinolyl)quinoline, 2-(4-isoquinolyl)quinoline, 3-(1-isoquinolyl)quinoline, 3-(3-isoquinolyl)quinoline, 3-(4-isoquinolyl)quinoline, 4-(1-isoquinolyl)quinoline, 4-(3-isoquinolyl)quinoline, and 4-(4-isoquinolyl)quinoline.

A solution containing an alkali metal, a reaction solvent, and the like that are the same as those described in item 1-1 above can be used in the method for manufacturing a nitrogen-containing bicyclic heterocyclic compound according to this embodiment.

In the method for manufacturing a nitrogen-containing bicyclic heterocyclic compound according to this embodiment, the nitrogen-containing heterocyclic compound represented by General Formula (IV) and the nitrogen-containing heterocyclic compound represented by General Formula (V) are used as starting materials (also referred to as "monomers" hereinafter), and the monomers are reacted with SD in the reaction solvent to manufacture the nitrogen-containing bicyclic heterocyclic compound represented by General Formula (VI). Reaction conditions such as a reaction temperature, a reaction time, and reaction atmosphere can be set to be the same as those described in item 1-1 above.

The usage amount of SD can be set as appropriate depending on the types and amounts of the monomers and the reaction solvent. When the monomers, namely the nitrogen-containing heterocyclic compound represented by General Formula (IV) and the nitrogen-containing heterocyclic compound represented by General Formula (V), are reacted with SD, it is preferable that 1 to 4 ml of the reaction solvent is used with respect to 1 mmol of the total substance amount of the monomers, and SD is reacted with the monomers in the reaction solvent in a condition where the ratio of the amount of the monomers to the amount of SD is 1:1 to 1:3. When different types of monomers are used, it is preferable to prepare the nitrogen-containing heterocyclic compound represented by General Formula (IV), the nitrogen-containing heterocyclic compound represented by General Formula (V), and SD such that a molar equivalent ratio therebetween is 1:1:1 to 1:1:3.

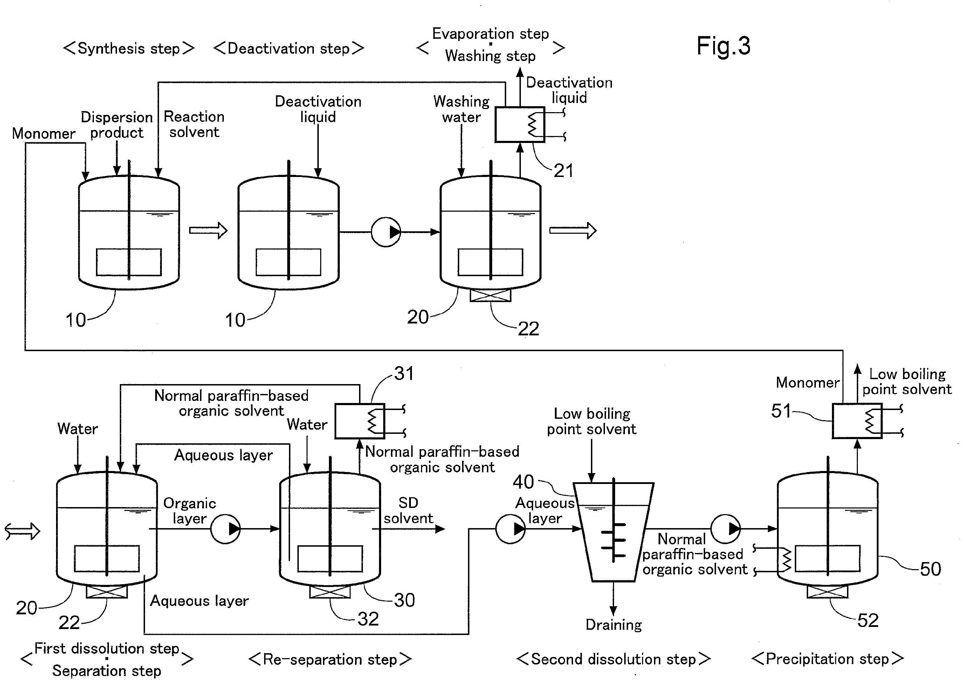

2. Purification of nitrogen-containing bicyclic heterocyclic compound As shown in FIG. 3, the method for manufacturing a nitrogen-containing bicyclic heterocyclic compound according to this embodiment further includes a deactivation step of deactivating the alkali metal remaining in the reaction solution containing the synthesized nitrogen-containing bicyclic heterocyclic compound, a first dissolution step of dissolving the dispersion solvent by adding a normal paraffin-based organic solvent to the reaction solution in which the alkali metal has been deactivated, and a separation step of separating an organic layer containing the normal paraffin-based organic solvent and an aqueous layer containing the nitrogen-containing bicyclic heterocyclic compound. The above-described synthesis step and the deactivation step are carried out in the same reaction vessel 10 under stirring. It should be noted that the synthesis step and the deactivation step may be performed in different vessels, and the stirring in the reaction vessel 10 may be omitted, but there is no particular limitation.

Alcohol or water is used as a deactivation liquid used in the deactivation step of deactivating the alkali metal remaining after the reaction. Lower alcohol such as isopropyl alcohol, methanol, or ethanol is preferably used as the alcohol, but higher alcohol may also be used, and there is no particular limitation. On the other hand, when water is used, it is preferable to carry out the deactivation step in an inert gas atmosphere that is filled with argon gas, nitrogen gas, or the like. It should be noted that when SD is used in the synthesis of the nitrogen-containing bicyclic heterocyclic compound, sodium is hydrogenated and is stable, and therefore, the deactivation step may be carried out in an air atmosphere when water is used. When alcohol is used, the deactivation step may be carried out in an inert gas atmosphere, and there is no particular limitation.

Hexane, heptane, octane, or the like is used as the normal paraffin-based organic solvent used in the first dissolution step. It is sufficient that the normal paraffin-based organic solvent dissolves the dispersion solvent but hardly dissolves the nitrogen-containing bicyclic heterocyclic compound. As a result, in the separation step, the dispersion solvent is contained in the organic layer, and the nitrogen-containing bicyclic heterocyclic compound is contained in the aqueous layer. Therefore, the insulation oil does not attach to the nitrogen-containing bicyclic heterocyclic compound, and the purity of the nitrogen-containing bicyclic heterocyclic compound can thus be improved.

Moreover, the method for manufacturing a nitrogen-containing bicyclic heterocyclic compound according to this embodiment further includes a second dissolution step of dissolving the nitrogen-containing bicyclic heterocyclic compound in a low boiling point solvent having a boiling point lower than the melting point of the nitrogen-containing bicyclic heterocyclic compound by adding the low boiling point solvent to the aqueous layer separated in the separation step, and a precipitation step of precipitating the nitrogen-containing bicyclic heterocyclic compound by evaporating the low boiling point solvent.

There is no particular limitation on the low boiling point solvent used in the second dissolution step as long as it dissolves the nitrogen-containing bicyclic heterocyclic compound and has a boiling point lower than the melting point of the nitrogen-containing bicyclic heterocyclic compound (for example, bipyrimidine has a melting point of about 112 to 116°C). Diethyl ether (with a boiling point of about 35°C), ethyl acetate (with a boiling point of about 77°C), hexane (with a boiling point of about 68°C), or the like is used as the low boiling point solvent, and diethyl ether is particularly preferable. In this embodiment, the nitrogen-containing bicyclic heterocyclic compound can be collected in the form of a solid merely by evaporating the low boiling point solvent in the precipitation step.

Hereinafter, a method for purifying a nitrogen-containing bicyclic heterocyclic compound will be described in detail. The reaction solution containing the nitrogen-containing bicyclic heterocyclic compound synthesized through the synthesis step and the deactivation step in the reaction vessel 10 is sent to a first dissolution vessel 20 using a pump or the like. Here, when normal paraffin oil (referred to as "SD solvent" hereinafter) is used as the dispersion solvent, THF is used as the reaction solvent, and ethanol is used as the deactivation liquid, the reaction solution mainly contains the SD solvent, THF, ethanol, metal alkoxide, sodium hydroxide, the unreacted monomers, and the synthesized nitrogen-containing bicyclic heterocyclic compound.

The reaction solution sent to the first dissolution vessel 20 is heated (e.g., at 80°C) for a predetermined period of time (e.g., 10 to 20 minutes) under reduced pressure using a heating unit 22 to evaporate THF (with a boiling point of about 66°C) and ethanol (with a boiling point of about 78°C). That is, this embodiment may further include an evaporation step of evaporating the reaction solvent by heating the reaction solution in which the alkali metal has been deactivated, the evaporation step being performed prior to the first dissolution step. It should be noted that the heating may be performed not under reduced pressure but under atmospheric pressure in the evaporation step, and there is no particular limitation.

Next, after evaporated THF and ethanol are cooled using a cooler 21 and liquefied, distillation treatment is performed using the difference between the boiling points of THF and ethanol, and THF is reused as the reaction solvent used in the synthesis step. Accordingly, a relatively large amount of the reaction solvent can be used efficiently, thus making it possible to reduce the manufacturing cost. It should be noted that the evaporated THF and ethanol may be discarded, and molecular sieves or the like may be used to adsorb and remove only alcohol. It is preferable to use alcohol whose boiling point is significantly different from that of THF as the alcohol serving as the deactivation liquid in order to facilitate the distillation of THF. When alcohol is adsorbed, it is preferable to select an alcohol such as ethanol or methanol whose corresponding molecular sieve can be easily obtained.

Next, water is added to the first dissolution vessel 20 under stirring to wash out the nitrogen-containing bicyclic heterocyclic compound, metal alkoxide, and the like that adhere to the wall surface of the first dissolution vessel 20. That is, this embodiment may further include a washing step of performing washing by adding water to the first dissolution vessel 20, the washing step being carried out prior to the first dissolution step. It should be noted that the washing step may also be carried out prior to the above-described evaporation step, and there is no particular limitation. In this case, a portion of the metal alkoxide reacts with water and thus decomposes into an extremely small amount of alcohol and sodium hydroxide in the washing step, thus making it possible to evaporate an extremely small amount of alcohol in the evaporation step.

Subsequently, the normal paraffin-based organic solvent is added to the reaction solution under stirring, and the SD solvent is dissolved by heating the mixture using a heating unit 22 to an extent that the organic solvent is not evaporated. Hexane (with a boiling point of about 69°C) is used as this organic solvent, but it is sufficient that a normal paraffin-based organic solvent whose boiling point is lower than that of the SD solvent is used. This is for the purpose of preventing the SD solvent from being mixed in the normal paraffin-based organic solvent when the normal paraffin-based organic solvent is evaporated and then reused. It should be noted that instead of water used in the washing step, the organic solvent used in the first dissolution step may be sprayed using a spray nozzle to wash the wall.

Although the evaporation step, the washing step, and the first dissolution step are carried out in the same first dissolution vessel 20, separate vessels may be used to carry out steps combined as appropriate or each step. When water is used as the deactivation liquid, the washing step may be carried out in the reaction vessel 10. It should be noted that, when water is used as the deactivation liquid, sodium hydroxide is produced instead of the metal alkoxide.

The reaction solution that has been subjected to the first dissolution step contains water, the SD solvent, the metal alkoxide, sodium hydroxide, the unreacted monomers, hexane, and the nitrogen-containing bicyclic heterocyclic compound.

Next, water is added to the first dissolution vessel 20, and an organic layer and an aqueous layer are separated through liquid-liquid separation. That is, this embodiment further includes a separation step of separating an organic layer and an aqueous layer, the separation step being carried out after the first dissolution step. As a result, the metal alkoxide decomposes into alcohol and sodium hydroxide, and the aqueous layer constituted by water, sodium hydroxide, the unreacted monomers, the nitrogen-containing bicyclic heterocyclic compound, and an extremely small amount of alcohol, and the organic layer constituted by the SD solvent, an extremely small amount of alcohol, and hexane, are separated through liquid-liquid separation. During the separation process, the first dissolution vessel 20 may be stirred, or the first dissolution vessel 20 may be shaken. A molecular sieve that adsorbs and removes water and alcohol may be used to remove an extremely small amount of alcohol contained in the aqueous layer. It should be noted that, when water is used as the deactivation liquid, alcohol is not produced, and therefore, the organic layer is constituted by the SD solvent and hexane. It should be noted that a separation vessel may be provided in addition to the first dissolution vessel 20 and used to separate the organic layer and the aqueous layer through liquid-liquid separation.

During the separation step, the unreacted monomers and the nitrogen-containing bicyclic heterocyclic compound are sometimes mixed in the organic layer. Therefore, in this embodiment, the organic layer in the first dissolution vessel 20 may be sent to a separation vessel 30 using a pump or the like. Water is added to this separation vessel 30 to further separate an organic layer and an aqueous layer through liquid-liquid separation. The unreacted monomers and the nitrogen-containing bicyclic heterocyclic compound are thereby separated therefrom into the aqueous layer, and this aqueous layer is sent to the first dissolution vessel 20 using a pump or the like and then reused. That is, this embodiment may further include a re-separation step of further adding water to the organic layer separated in the separation step and returning the nitrogen-containing bicyclic heterocyclic compound to the first dissolution vessel 20.

Next, the organic layer is heated (e.g., at 60°C) under reduced pressure for a predetermined period of time using a heating unit 32 to evaporate an extremely small amount of alcohol and hexane, and the SD solvent is discharged and discarded. The evaporated hexane may be cooled using a cooler 31 and liquefied, and then reused as the normal paraffin-based organic solvent used in the first dissolution step. Accordingly, an organic solvent needed in a relatively large amount can be saved, thus making it possible to reduce the manufacturing cost. It should be noted that hexane may be discarded, and there is no particular limitation.

On the other hand, after the separation step, the aqueous layer is sent to a second dissolution vessel 40 using a pump or the like. Diethyl ether serving as the low boiling point solvent is added to this second dissolution vessel 40 to dissolve the nitrogen-containing bicyclic heterocyclic compound and the unreacted monomers (second dissolution step). It is preferable to carry out this second dissolution step while the aqueous layer is caused to flow under stirring. It is particularly preferable to stir the aqueous layer using a PV mixer having discontinuous multi-stage tilted paddle blades because the concentration of the nitrogen-containing bicyclic heterocyclic compound is made uniform. Then, the organic layer is allowed to stand and subjected to separation, and water and sodium hydroxide are drained out.

Next, the nitrogen-containing bicyclic heterocyclic compound and the unreacted monomers dissolved in diethyl ether are sent to a precipitation vessel 50 using a pump or the like. In this precipitation vessel 50, the reaction solution is heated using a heating unit 52 at a temperature that is higher than or equal to the boiling point of the unreacted monomers (for example, pyrimidine has a boiling point of about 123°C). As a result, the nitrogen-containing bicyclic heterocyclic compound is precipitated, and diethyl ether and the unreacted monomers are evaporated. The precipitated nitrogen-containing bicyclic heterocyclic compound is collected as a product.

Subsequently, after the evaporated diethyl ether and unreacted monomers are cooled using a cooler 51 and liquefied, distillation treatment is performed using a difference between the boiling points of diethyl ether and the monomers, and the unreacted monomers are reused as the monomers in the synthesis step. Accordingly, the unreacted monomers can be used efficiently, thus making it possible to reduce the manufacturing cost. It should be noted that the evaporated diethyl ether and the unreacted monomers may be discarded, and diethyl ether may be reused in the second dissolution vessel 40.

Moreover, in the precipitation vessel 50, the reaction solution may be heated at a temperature that is higher than or equal to the boiling point of diethyl ether (about 35°C) and lower than or equal to the boiling point of the unreacted monomers (for example, pyrimidine has a boiling point of about 123°C). In this case, it is preferable to remove the unreacted monomers present in a liquid form through washing treatment in order to collect the nitrogen-containing bicyclic heterocyclic compound.

Other embodiments
(1) In the above-described purification of the nitrogen-containing bicyclic heterocyclic compound, a recrystallization step may be carried out after the low boiling point solvent is evaporated in the precipitation step. In this case, a concentration step of concentrating the nitrogen-containing bicyclic heterocyclic compound by adding a recrystallization solvent such as ethanol, a cooling step of cooling the recrystallization solvent containing the nitrogen-containing bicyclic heterocyclic compound, and a filtration step of collecting the nitrogen-containing bicyclic heterocyclic compound through filtration are included, the cooling step being carried out after the concentration step and the filtration step being carried out after the cooling step. After the concentration step, the nitrogen-containing bicyclic heterocyclic compound in such an amount that corresponds to an amount exceeding saturation solubility is recrystallized. On the other hand, the unreacted monomers remain dissolved in the recrystallization solvent. Therefore, in the filtration step, only the nitrogen-containing bicyclic heterocyclic compound in which no other impurities are contained can be collected efficiently.
(2) The order of the steps in the above-described purification of the nitrogen-containing bicyclic heterocyclic compound may be changed as appropriate, or some of them may be omitted, without departing from the spirit or essential characteristics of the present invention.

### Examples

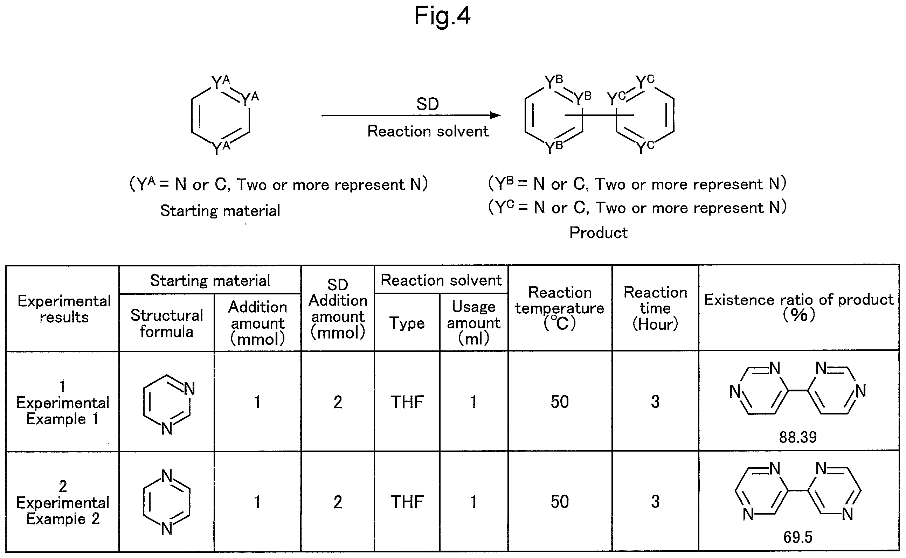

Hereinafter, the present invention will be described in detail by use of examples, but the present invention is not limited to these examples. FIG. 4 summarizes the conditions and the results of the investigations in the following examples. The existence ratio of a product is a ratio to all the products shown as a percentage (%). It should be noted that a dispersion product obtained by dispersing minute particles of sodium metal in normal paraffin oil was used as SD in the following examples, and the substance amount of SD was a value in terms of sodium metal contained in SD.

### Example 1: Production of 4,4'-bipyrimidine through coupling of pyrimidine

In 1 ml of THF, 1 mmol of pyrimidine was dissolved, and 2 mmol of SD was added thereto and reacted therewith at 50°C for 3 hours. The reaction was performed in an atmosphere. As a result, as shown in Reaction Formula (1) below, 4,4'-bipyrimidine was obtained as a main product.

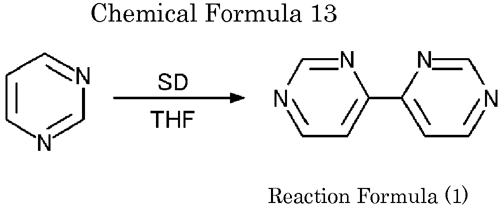

### Example 2: Production of 2,2'-bipyrazine through coupling of pyrazine

In 1 ml of THF, 1 mmol of pyrazine was dissolved, and 2 mmol of SD was added thereto and reacted therewith at 50°C for 3 hours. The reaction was performed in an atmosphere. As a result, as shown in Reaction Formula (2) below, 2,2'-bipyrazine was obtained as a main product.

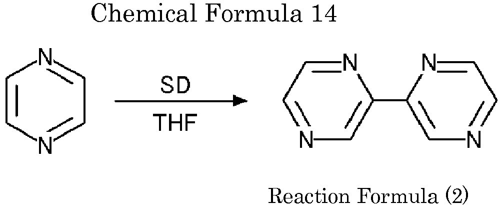

### Industrial Applicability

The present invention can be applied to all the technical fields in which a nitrogen-containing bicyclic heterocyclic compound such as a bipyrimidine compound, bipyrazine compound, or biquinoline compound is used, and particularly to manufacturing of organic EL materials, pharmaceuticals, agricultural chemicals, dyes, and the like, basic research thereon, and a reaction for the reduction of carbon dioxide.

## Claims

1. A method for manufacturing a nitrogen-containing bicyclic heterocyclic compound, comprising a synthesis step of synthesizing a nitrogen-containing bicyclic heterocyclic compound represented by General Formula (III) below by reacting, in a reaction solvent, a nitrogen-containing heterocyclic compound represented by General Formula (I) below and a nitrogen-containing heterocyclic compound represented by General Formula (II) below with a solution containing an alkali metal: 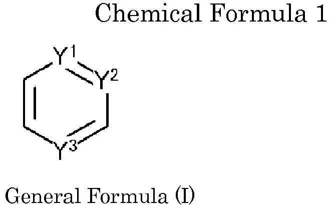 where Y¹, Y², and Y³ in General Formula (I) independently represent a nitrogen atom or a carbon atom, at least two of which represent a nitrogen atom; 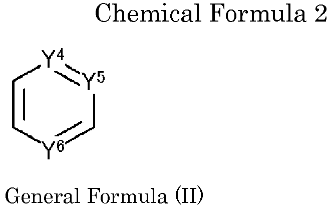 where Y⁴, Y⁵, and Y⁶ in General Formula (II) independently represent a nitrogen atom or a carbon atom, at least one of which represent a nitrogen atom; and 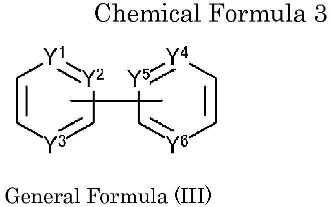 where Y¹, Y², and Y³ in General Formula (III) independently represent a nitrogen atom or a carbon atom, at least two of which represent a nitrogen atom, and are respectively the same as Y¹, Y², and Y³ in General Formula (I), while Y⁴, Y⁵, and Y⁶ in General Formula (III) independently represent a nitrogen atom or a carbon atom, at least one of which represents a nitrogen atom, and are respectively the same as Y⁴, Y⁵, and Y⁶ in General Formula (II).

2. The method for manufacturing a nitrogen-containing bicyclic heterocyclic compound according to claim 1, wherein the Y¹ represents a carbon atom, the Y² and the Y³ represent a nitrogen atom, the Y⁴ represents a carbon atom, and the Y⁵ and the Y⁶ represent a nitrogen atom.

3. The method for manufacturing a nitrogen-containing bicyclic heterocyclic compound according to claim 1, wherein the Y¹ and the Y³ represent a nitrogen atom, the Y² represents a carbon atom, the Y⁴ and the Y⁶ represent a nitrogen atom, and the Y⁵ represents a carbon atom.

4. A method for manufacturing a nitrogen-containing bicyclic heterocyclic compound, comprising a synthesis step of synthesizing a nitrogen-containing bicyclic heterocyclic compound represented by General Formula (VI) below by reacting, in a reaction solvent, a nitrogen-containing heterocyclic compound represented by General Formula (IV) below and a nitrogen-containing heterocyclic compound represented by General Formula (V) below with a solution containing an alkali metal: 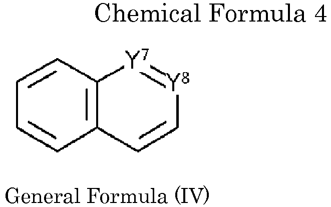 where Y⁷ and Y⁸ in General Formula (IV) independently represent a nitrogen atom or a carbon atom, one of which represents a nitrogen atom and the other of which represents a carbon atom; 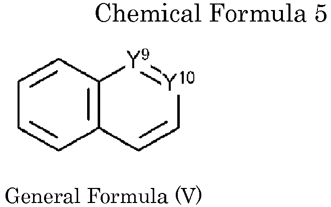 where Y⁹ and Y¹⁰ in General Formula (V) independently represent a nitrogen atom or a carbon atom, one of which represents a nitrogen atom and the other of which represents a carbon atom; and 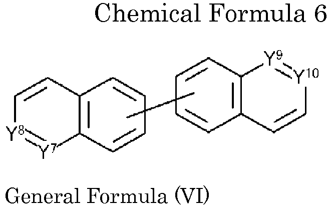 where Y⁷ and Y⁸ in General Formula (VI) independently represent a nitrogen atom or a carbon atom, one of which represents a nitrogen atom and the other of which represents a carbon atom, and are respectively the same as Y⁷ and Y⁸ in General Formula (IV), while Y⁹ and Y¹⁰ in General Formula (VI) independently represent a nitrogen atom or a carbon atom, one of which represents a nitrogen atom and the other of which represents a carbon atom, and are respectively the same as Y⁹ and Y¹⁰ in General Formula (V).

5. The method for manufacturing a nitrogen-containing bicyclic heterocyclic compound according to any one of claims 1 to 4,
wherein the solution containing an alkali metal is a dispersion product obtained by dispersing the alkali metal in a dispersion solvent, and
the method comprises:
a deactivation step of deactivating the alkali metal remaining in the reaction solution containing the synthesized nitrogen-containing bicyclic heterocyclic compound;
a first dissolution step of dissolving the dispersion solvent by adding a normal paraffin-based organic solvent to the reaction solution in which the alkali metal has been deactivated; and
a separation step of separating an organic layer containing the normal paraffin-based organic solvent and an aqueous layer containing the nitrogen-containing bicyclic heterocyclic compound.

6. The method for manufacturing a nitrogen-containing bicyclic heterocyclic compound according to claim 5, further comprising:
a second dissolution step of dissolving the nitrogen-containing bicyclic heterocyclic compound in a low boiling point solvent having a boiling point lower than the melting point of the nitrogen-containing bicyclic heterocyclic compound by adding the low boiling point solvent to the aqueous layer; and
a precipitation step of precipitating the nitrogen-containing bicyclic heterocyclic compound by evaporating the low boiling point solvent.
